# EUROPÄISCHE PATENTSCHRIFT

(11) **EP 1 262 776 B1**
(45) Veröffentlichungstag und Bekanntmachung des Hinweises auf die Patenterteilung: **01.03.2006**
(21) Anmeldenummer: 02011919.4
(22) Anmeldetag: 29.05.2002
(51) Int. Cl.: G01N 33/543, G01N 33/574, G01N 33/50

(54) **Verfahren zum quantitativen Nachweis vitaler ephithelialer Tumorzellen in einer Körperflüssigkeit**
Method for the quantitative detection of vital epithelial tumour cells in a body fluid
Procédé pour la détermination quantitative de cellules tumorales épithéliales vivantes dans un fluide corporel

(30) Priorität: 02.06.2001 DE 10127079
(43) Veröffentlichungstag der Anmeldung: 04.12.2002
(73) Patentinhaber: Pachmann, Ulrich, 95448 Bayreuth (DE); Pachmann, Katharina, 95448 Bayreuth (DE)
(72) Erfinder: Pachmann, Ulrich, 95448 Bayreuth (DE); Pachmann, Katharina, 95448 Bayreuth (DE)
(74) Vertreter: Gassner, Wolfgang

(56) Entgegenhaltungen:
- WO-A-99/41613
- US-A- 5 751 839
- PACHMANN K ET AL: "REAL TIME MONITORING OF THE EFFICACY OF ADJUVANT THERAPY IN BREAST CANCER QUANTIFYING THE REDUCTION OF CIRCULATING TUMOR CELLS BY MAINTRAC (LASER SCANNING CYTOMETRY OF MAGNETIC BEAD ENRICHED CELLS)" BREAST CANCER RESEARCH AND TREATMENT, NIJHOFF, BOSTON, US, Bd. 64, Nr. 1, November 2000 (2000-11), Seite 96 XP009017863 ISSN: 0167-6806
- PACHMANN R N ET AL: "DETECTION OF BREAST CANCER CELLS IN BONE MARROW AND PERIPHERAL BLOOD WITH MAGNETIC BEAD ENRICHMENT AND LASER SCANNING CYTOMETRY (MAINTRAC)" BREAST CANCER RESEARCH AND TREATMENT, NIJHOFF, BOSTON, US, Bd. 64, Nr. 1, November 2000 (2000-11), Seite 97 XP009017864 ISSN: 0167-6806
- "HEA-FITC" MACS, [Online] XP002258639 Gefunden im Internet: <URL:www.miltenyibiotec.com> [gefunden am 2003-10-21]
- "Magnetic Cell Sorting and Separation of Biomolecules" 1999, MILTENYI BIOTEC * Seite 71 *

## Beschreibung

Die Erfindung betrifft ein Verfahren zum quantitativen Nachweis vitaler, epithelialer Tumorzellen in einer Körperflüssigkeit.

Die Erfindung betrifft allgemein das Gebiet der Indikation solider Tumoren. Bekanntlich ist die Metastasierung bei soliden Tumoren der Hauptgrund für hohe Krebssterblichkeit. Sie wird durch Zellen verursacht, die in den Lymphknoten disseminiert werden und/oder im peripheren Blut zirkulieren. Von den zirkulierenden Tumorzellen kann unter Umständen ein Teil in entfernte. Kompärtimente gelangen, wo sie erneut zu wachsen beginnen. Derartige Kompärtimente sind bei einigen Tumoren bekannt. Bei Brustkrebs und Dickdarmkrebs ist ein solches Kompartiment das Knochenmark. Die Häufigkeit der Tumorzellen in bezug auf normale Knochenmarkszellen beträgt höchstens 10 ⁻³ bis 10⁻⁷ Tumorzellen/normale Knochenmarkszellen. Um Proben für eine Knochenmarksdiagnostik zu erhalten, ist ein spezieller Eingriff in Verbindung mit oder nach einer Operation erforderlich. Eine regelmäßige Kontrolle würde eine Wiederholung eines derartigen Eingriffs erfordern. Wegen der damit verbundenen Unannehmlichkeiten für den Patienten sowie des Kosten- und Zeitaufwands ist man bestrebt, die Anzahl operativer Eingriffe möglichst gering zu halten.

Eine weitere Möglichkeit bietet eine Untersuchung des wesentlich leichter zugänglichen peripheren Blutes. Dabei tritt allerdings das Problem auf, daß darin nachweisbare Tumorzellen nur in äußert geringer Anzahl vorhanden sind. Erschwerend kommt hinzu, daß die im peripheren Blut zirkulierenden Tumorzellen das Transplantat bei einer Hochdosis-Chemotherapie oder einer autologen peripheren Blutstammzellentransplantation kontaminieren können. Es sind deshalb hochempfindliche Systeme notwendig, um eine derart geringe Anzahl an restlichen Tumorzellen nachzuweisen.

Das derzeit empfindlichste Nachweisverfahren ist die Polymerase-Kettenreaktion (PCR). Bei hämatologischen Malignomen zeigt die PCR von Gensequenzen, die mit dem Tumor assoziert sind, eine hohe Empfindlichkeit beim Nachweis einer geringen Zahl an Tumorzellen. Allerdings ist bei soliden Tumoren die PCR mit Problemen hinsichtlich der Anwendbarkeit, der Spezifität und des klinischen Einflusses verbunden. Als weiteres Verfahren kann auch die gewebe- und reifungsabhängige Expression der Oberflächen- oder intrazellulären Antigene genutzt werden, um aberrante Zellen vom normalem Gewebe immunlogisch zu unterscheiden. Die Häufigkeit von 10⁻³ bis 10⁻⁸, mit der Tumorzellen aus soliden Tumoren normalerweise in peripherem Blut zu erwarten sind, erfordert aber die Prüfung einer hohen Anzahl negativer Zellen, um eine positive Zelle bzw. eine Tumorzelle zu finden. Ein derartiger immunologischer Nachweis der Tumorzellen muß mit Hilfe eines Mikroskops vorgenommen werden. Er ist mit einem sehr hohen Aufwand verbunden. Dabei können Tumorzellen aufgrund ihrer geringen Anzahl übersehen werden. Die Genauigkeit eines solchen Nachweisverfahrens ist vergleichsweise gering. Auch die Verwendung von Bildanalyseverfahren verbessert die Sensitivität und die Geschwindigkeit nur unwesentlich.

Aus der WO 99/41613 ist ein Verfahren zum quantitativen Nachweis von epithelialen Zellen im peripheren Blut von Krebspatienten bekannt. Dabei werden Zellen, die epitheliale Antigene tragen, aus peripherem Blut über magnetische Partikel angereichert und mit gegen epitheliale Antigene gerichteten fluoreszentmarkierten Antikörpern angefärbt, um sie anschließend mittels Durchflußzytometrie quantitativ zu erfassen. Die Zahl der erfassten epithelialen Zellen wird auf 10 ml Blut normalisiert.

Aus Rinas, N. et al., Breast Cancer Research and Treatment, NIJHOFF, Boston, US, Band 64, Nr. 1, November 2000 (2000-11), Seite 97 ist es bekannt, im Blut zirkulierende Brustkrebszellen mittels magnetischer Partikel anzureichern und mittels Laser-Scanning-Cytometrie zu quantifizieren. Dazu wurden an magnetische Partikel gekoppelte und mit FITC konjugierte antihumane epitheliale Antikörper verwendet.

Aus Pachmann K. et al., Breast Cancer Research and Treatment, NIJHOFF, Boston, US, Band 64, Nr. 1, November 2000 (2000-11), Seite 96 ist es bekannt, aus peripherem Blut von Brustkrebspatientinnen mittels magnetischer Partikel Zellen anzureichern, welche das epitheliale Antigen tragen und die Zellen mittels eines Fluorochrom-markierten Antikörpers für epitheliale Antigene zu färben. Die Zellen wurden dann in einem Laser-Scanning-Cytometer gemessen.

Bei bekannten Verfahren wird die Anzahl der Tumorzellen in bezug auf die Anzahl aller Zellen, beispielsweise der Leukozyten, ermittelt. Die Anzahl der Leukozyten kann aber zum Beispiel bei einer Hochdosis-Chemotherapie stark schwanken, so daß die Anzahl der Tumorzellen in bezug auf die Anzahl von Leukozyten nur bedingt aussagefähig ist.

Aufgabe der Erfindung ist es, die Nachteile nach dem Stand der Technik zu beseitigen. Es soll insbesondere ein verbessertes Verfahren zum quantitativen Nachweis vitaler epithelialer Tumorzellen in einer Körperflüssigkeit angegeben werden, dessen Genauigkeit und Geschwindigkeit die bisher bekannter Verfahren übertrifft.

Diese Aufgabe wird durch die Merkmale des Anspruchs 1 gelöst. Zweckmäßige Ausgestaltungen der Erfindung ergeben sich aus den Merkmalen der Ansprüche 2 bis 10.

Nach Maßgabe der Erfindung ist ein Verfahren zum quantitativen Nachweis vitaler epithelialer Tumorzellen in einer bestimmten Menge einer Körperflüssigkeit mit folgenden Schritten vorgesehen:
a) Markieren der vitalen epithelialen Tumorzellen durch Zugabe von gegen humanes epitheliales Antigen gerichteten Antikörpern, die an magnetische Partikel gebunden sind, zu der Körperflüssigkeit, wobei das humane epitheliale Antigen ein Antigen ist, welches durch den monoklonalen Antikörper HEA 125 erkannt wird,
b) Markieren der vitalen epithelialen Tumorzellen durch Zugabe von gegen das humane epitheliale Antigen gerichteten Antikörpern, die an ein Fluorochrom gebunden sind, zu der Körperflüssigkeit,
c) Magnetische Anreicherung der vitalen epithelialen Tumorzellen,
d) Immobilisieren der so erhaltenen Suspension auf einem Trägermaterial durch selbständiges Anhaften der vitalen Tumorzellen,
e) Erfassen der vitalen epithelialen Tumorzellen mittels Laser-Scanning-Cytometrie und Berechnen der Anzahl dieser Zellen in bezug auf die Menge der bereitgestellten Körperflüssigkeit.

Das vorgeschlagene Verfahren bietet die Möglichkeit, die Anzahl von vitalen epithelialen Tumorzellen direkt in einer Körperflüssigkeit, wie beispielsweise Blut, Knochenmark, Knochenmark-Aspirat, Transsüdat, Exsudat, Lymphe, Apheresat, Aszites, Urin, Speichel und Drainageflüssigkeiten aus Wundsekreten, zu bestimmen. Die eingesetzten Antikörper binden spezifisch an vitale tumorverdächtige Zellen. Vitale Tumorzellen können damit von toten Tumorzellen getrennt werden. Die Anzahl der vitalen Tumorzellen kann in bezug auf das Volumen der eingesetzten Körperflüssigkeit angegeben werden. Das erfindungsgemäße Verfahren liefert standardisierte Werte. Überdies kann das Trägermaterial, auf dem die untersuchte Körperflüssigkeit nach Markierung, Anreicherung und Trennung aufgetragen wird, zu Dokumentationszwecken aufbewahrt werden, so daß es für eine spätere Begutachtung und weitere Charakterisierung zur Verfügung steht. Bei den bisherigen Verfahren kann demgegenüber zumeist nur ein Meßprotokoll aufbewahrt werden.

Mit dem Verfahren der vorliegenden Erfindung können sehr geringe Mengen an Tumorzellen in einer Körperflüssigkeit nachgewiesen werden. Zu Testzwecken wurden beispielsweise zehn Tumorzellen zu 20 ml Vollblut gegeben. Diese zehn Tumorzellen konnten mit dem erfindungsgemäßen Verfahren vollständig nachgewiesen werden. Dieses Ergebnis ist mit dem vergleichbar, das mittels PCR erreicht werden kann, wobei jedoch die mit diesem verfahren verbundenen Nachteile vermieden werden können. Das erfindungsgemäße Verfahren erlaubt die quantitative Bestimmung vitaler epithelialer Tumorzellen in einer Körperflüssigkeit.

Zweckmäßigerweise wird die Körperflüssigkeit, insbesondere peripheres Blut, vor der Markierung der vitalen epithelialen Tumorzellen mit Antikörpern lysiert, um beispielsweise Erythrozyten abzutrennen. Die erhaltene Suspension wird anschließend zentrifugiert, der Überstand abgetrennt und verworfen. Es ist nicht erforderlich, daß alle Erythrozyten entfernt werden, da diese das Verfahren nicht beeinflussen.

In einer Variante der Erfindung werden die Tumorzellen vor der magnetischen Anreicherung sowohl mit gegen das humane epitheliale Antigen gerichteten Antikörpern, die an magnetische Partikel gebunden sind ("magnetic beads" oder "Microbeads"), als auch mit gegen das humane epitheliale Antigen gerichteten Antikörpern, die an ein Fluorochrom gebunden sind, markiert. Die magnetischen Partikel haben vorzugsweise einen Durchmesser, der kleiner als 70 nm ist. Die Tumorzellen tragen über die Antigen-Antikörper-Bindung sowohl magnetische Partikel als auch einen Fluoreszenzfarbstoff. Die Tumorzellen werden anschließend durch magnetische Zellseparation angereichert, indem sie z.B. auf eine Säule gegeben werden, die sich in einem starken, z.B. durch einen Permanentmagnet gebildeten, Magnetfeld befindet. Die Zellen der Körperflüssigkeit, an die keine magnetischen Partikel gebunden sind, werden aus der Säule herausgespült. Die markierten Zellen verbleiben wegen der Wirkung des Magnetfelds in der Säule. Nach dem Entfernen des Magnetfelds können die in der Säule verbliebenen Tumorzellen ausgespült werden.

Alternativ kann die magnetische Zellseparation erfolgen, bevor die vitalen epithelialen Tumorzellen durch Zugabe von gegen das humane epitheliale Antigen gerichteten Antikörpern, die an ein Fluorochrom gebunden sind, markiert werden. Dazu ist es ausreichend, wenn die Tumorzellen mit den magnetischen Partikeln markiert werden.

Zweckmäßigerweise wird vor dem Markieren der vitalen epithelialen Tumorzellen ein FcR-Blockierungsreagenz zu der Körperflüssigkeit zugesetzt.

Als Antikörper werden vorzugsweise gegen das humane epitheliale Antigen gerichtete Antikörper (HEA) verwendet, die von der Maus stammen. Als Fluorochrom wird vorzugsweise Fluoresceinisothiocyanat (FITC) eingesetzt.

Nach der Anreicherung und Einfärbung der vitalen epithelialen Tumorzellen wird die Zellsuspension auf einen Träger aufgebracht. Dabei handelt es sich zweckmäßigerweise um einen Objektträger aus Glas, der vorzugsweise mit Poly-L-Lysin beschichtet ist.

Die Anzahl der Tumorzellen auf dem Träger bzw.Trägermaterial wird mittels Laser-Scanning-Cytometrie bestimmt. Zur Konturierung der auf dem Trägermaterial befindlichen Zellen wird zweckmäßigerweise das Vorwärtsstreulicht (forward scatter) als Schwellwertparameter bei einer geringen Vergrößerung genutzt. Die Hintergrundfluoreszenz kann dynamisch bestimmt werden, um die maximale Fluoreszenzintensität und/oder die Gesamtfluoreszenz durch Integration über jede Zelle zu bestimmen. Das ermöglicht es, Veränderungen der Hintergrundfluoreszenz zu korrigieren, so daß die Fluoreszenz für jede Zelle unter den gleichen Bedingungen berechnet werden kann und äquivalente Ergebnisse für jede Zelle erhalten werden. Die grüne Fluoreszenz der FITC-HEA-markierten Zellen wird vorzugsweise unter Verwendung eines 530/30 nm-Bandpaßfilters erfaßt.

Die angereicherten und FITC-markierten Zellen (FITC-positive Zellen) können außerdem mit einem weiteren Fluoreszenzfarbstoff angefärbt werden. Beispielsweise kann die DNA der Zellkerne mit einem DNA-spezifischen Farbstoff wie Propidiumiodid angefärbt werden. Die rote Fluoreszenz der so angefärbten Zellen wird ebenfalls mittels Laser-Scanning-Cytometrie unter Verwendung eines 625/28 nm-Bandpaßfilters erfaßt. Als Schwellwertparameter zur Konturierung dient das Vorwärtsstreulicht. Die gemessene rote und grüne Fluoreszenz werden miteinander verglichen und die Anzahl der positiven Ereignisse ermittelt.

Die Anzahl an markierten positiven Zellen ist im Vergleich zu nach dem im Stand der Technik bekannten Verfahren aufgrund der Anreicherung pro Volumen besonders hoch, so daß die Geschwindigkeit und die Genauigkeit des erfindungsgemäßen Verfahrens deutlich höher sind. Die Anzahl der gefundenen markierten Zellen wird dann auf das ursprünglich eingesetzte Volumen an Körperflüssigkeit zurückgerechnet. Das erlaubt es zu verschiedenen Zeitpunkten ermittelte Nachweisergebnisse zu vergleichen. Es kann rasch der Erfolg z.B. einer Tumortherapie erkannt werden.

Die Morphologie der erfaßten positiven Zellen kann anschließend durch hämatologische Färbeverfahren wie die May-Grünwald-Färbung bestimmt werden.

Das erfindungsgemäße Verfahren erlaubt vorteilhafterweise die Durchführung mehrerer quantitativer Nachweise hintereinander. Die Zellen können quantitativ im Hinblick auf verschiedene Parameter untersucht werden. Dazu können die Zellen nach einer ersten quantitativen Auswertung z.B. mit einer einen zweiten Nachweisstoff, z.B. einen Fluoreszenzfarbstoff, enthaltenden Lösung beaufschlagt werden, welcher z.B. spezifisch für Malignität ist. Die Koordinaten der Zellen sind bereits von der Durchführung des ersten quantitativen Nachweises bekannt und im Computer gespeichert. Es kann jede Zelle sofort aufgefunden und deren Reaktion gegenüber weiteren Nachweissubstanzen erfaßt und quantitativ ausgewertet werden. Als weitere Nachweisverfahren können insbesondere FISH oder TUNEL durchgeführt und quantitativ ausgewertet werden.

Nachfolgend wird die Erfindung anhand von Ausführungsbeispielen näher erläutert.

### Beispiel 1

In diesem Beispiel wird ein Verfahren zum Nachweis von epithelialen Tumorzellen in Blut beschrieben, bei dem die magnetische Anreicherung der vitalen epithelialen Tumorzellen nach der Markierung der vitalen epithelialen Tumorzellen durch Zugabe von gegen das humane epitheliale Antigen gerichteten Antikörpern, an die ein Fluorochrom gebunden ist, erfolgt.

### a. Herstellen einer Verdünnungsreihe von Tumorzellen enthaltendem Vollblut

Peripheres Blut wurde mit verschiedenen Anteilen an Tumorzellen gemischt. Die Tumorzellen stammten aus einer Brustkrebszellinie (SK Br2-artig). Die Leukozyten des peripheren Blutes und die Tumorzellen wurden gezählt. In einem ersten Verdünnungsschritt wurden 6 x 10⁵ Tumorzellen mit 100 µl Vollblut, das 6 x 10⁵ Leukozyten enthielt, gemischt. Im nächsten Verdünnungsschritt wurden jeweils 6 x 10⁴ Tumorzellen und 6 x 10³ Tumorzellen zu 100 µl Blut gegeben. Im anschließenden Verdünnungsschritt wurden 6 x 10³ und 6 x 10² Tumorzellen zu 1 ml Blut sowie 6 x 10² und 60 Tumorzellen zu 10 ml oder 20 ml Blut gegeben, wobei im letzten Fall eine Verdünnung von 5 Tumorzellen auf 10⁷ normale Zellen erhalten wurde.

### b. Magnetische Anreicherung und Anfärbung

Zur Trennung an einer magnetischen Säule wurden 400 µl mit bis zu 5 x 10⁷ Zellen aus der in lit. a. erhaltenen Verdünnungsreihe mit 100 µl Blockierungsreagens 30 min in der Kälte inkubiert. Die Zellen wurden dann mit magnetischen Partikeln, an die gegen das humane epitheliale Antigen gerichtete Antikörper gebunden sind, (100 µl HEA-Microbeads, Miltenyi) behandelt, 15 min inkubiert und 10mal mit dem Markierungspuffer gewaschen. Die Säulen, an denen ein Magnet angebracht ist, wurden gewaschen und die magnetischen Partikel in den Säulen gesammelt. Die negativen Zellen wurden dann durch Spülen mit 5mal 500 µl Puffer eluiert und die Säulen vom Magnet getrennt. Die in den Säulen verbliebenen Zellen wurden mit zusätzlichem Puffer herausgespült und mit 50 µl FITCkonjugierten gegen das humane epitheliale Antigen gerichteten Mäuseantikörpern (HEA-FITC-Antikörper, Miltenyi) angefärbt.

### c. Immobilisierung der Zellen und Bestimmung der Anzahl der Zellen

Die Zellen wurden auf Objektträger aufgetragen. Nach dem Auftragen von 100 µl der Zellsuspension hafteten die vitalen Zellen nach 10 bis 15 min auf der Oberfläche des Objektträgers. Für optimale Messungen muß eine Einzelzellsuspension auf dem Objektträger aufgetragen werden, wobei die Zellen einen Abstand untereinander aufweisen, der etwa dem 2- bis 3fachen des Durchmessers einer Zelle entspricht.

Die anhaftenden Zellen wurden unter Verwendung eines Laser-Scanning-Cytometers (LSC Compucyte Corp.) gemessen. Die Konturierung der auf dem Objektträger haftenden Zellen wurde unter Verwendung des Vorwärtsstreulichtes (forward scatter) als Schwellwertparameter bei einer zwanzigfachen Vergrößerung durchgeführt. Die Hintergrundfluoreszenz wurde dynamisch bestimmt, um sowohl die maximale Fluoreszenzintensität als auch die Gesamtfluoreszenz auf Basis einer Zelle zu berechnen. Dies ermöglicht es, die Schwankungen der Hintergrundfluoreszenz zu korrigieren, so daß die Berechnung der Fluoreszenz für alle Zellen gleichwertig erfolgt. Die grüne Fluoreszenz der positiven HEA-FITC-Zellen wurde mit einem 530/30 nm-Bandpaßfilter erfaßt und mit einem Photomultiplier verstärkt.

Anschließend wurden die Zellen auf den Objektträgern zentrifugiert und in eine 1 mg/ml Propidiumiodid enthaltende PBS-Lösung getaucht. Der Schwellwert für die Konturierung der Zellen basierte ebenfalls auf dem Vorwärtsstreulicht. Die rote Fluoreszenz wurde mit einem 625/30 nm Bandpaßfilter erfaßt und mit einem zweiten Photomultiplier verstärkt. Die rote und grüne Fluoreszenz wurden mittels eines Computerprogrammes (WinCyte, Compucyte Corporation) angeglichen und als Streulichtdiagramme, Histogramme, Prozentwerte und Mittelwerte der FITC-positiven und FITC-negativen Zellen dargestellt, wobei die Berechnung nur auf dem Bereich beruht, der Einzelzellen umfaßt.

### d. Ergebnis

Die Korrelation zwischen der berechneten Anzahl von Tumorzellen in einer Probe der Verdünnungsreihe und der mittels dem Verfahren ermittelten Anzahl war sehr hoch (> 0,99). Sogar bei der 10⁻⁸-fachen Verdünnung konnten 50 von 60 Zellen nachgewiesen werden.

### Beispiel 2

In diesem Beispiel wird ein Verfahren zum Nachweis von epithelialen Tumorzellen in Blut beschrieben, wobei die magnetische Anreicherung der vitalen epithelialen Tumorzellen vor der Markierung der vitalen epithelialen Tumorzellen durch Zugabe von gegen das humane epitheliale Antigen gerichteten Antikörpern, die an ein Fluorochrom gebunden sind, erfolgt.

### a. Markierung der Tumorzellen

20 ml peripheres Blut wurde mit 40 ml Erylysispuffer (155 mM NH₄Cl, 10 mM KHCO₃, 1 mM Na₂-EDTA) versetzt und in der Kälte 7 min bei 2000 rpm zentrifugiert. Der Überstand wurde verworfen und das Sediment in PBS-EDTA-Lösung (50 ml PBS mit 200 µl 0,5 M EDTA) aufgenommen, so daß das Gesamtvolumen 900 µl betrug. Zu 300 µl dieses Gemisches wurden 100 µl FcR-Blockierungsreagenz (Miltenyi), 100 µl der magnetischen Partikel, an die gegen das humane epitheliale Antigen gerichtete Antikörper gebunden sind, (100 µl HEA-Microbeads, Miltenyi) zugegeben und gemischt. Anschließend wurden zu dieser Suspension 50 µl FITC-konjugierte gegen das humane epitheliale Antigen gerichtete Mäuseantikörper (HEA-FITC-Antikörper, Miltenyi) zugegeben und 15 min in der Kälte inkubiert.

### b. Magnetische Anreicherung

Die Separationssäulen wurden in den Magneten (ctaMACS, Miltenyi) gestellt und zweimal mit 500 µl PBS-EDTA-Lösung gewaschen. Die in a. erhaltene Suspension wurde mit 500 µl PBS-EDTA-Lösung aufgeschwemmt und auf die Säule gegeben. Die Säule wurde dreimal mit 500 µl PBS-EDTA-Lösung gewaschen und aus dem Magneten entnommen. Die Säule wurde anschließend mit 200 µl PBS-EDTA-Lösung gespült, um die markierten Zellen aufzunehmen.

### c. Immobilisierung der Zellen und Bestimmung der Anzahl der Zellen

Auf einen mit Poly-L-Lysin beschichteten Objektträger (Labor Schubert) wurden 100 µl der bei lit. b. erhaltenen Lösung aufgetragen und gleichmäßig verteilt. Die Anzahl der Tumorzellen wurde wie in Beispiel 1, lit. c bestimmt und die Anzahl in bezug auf das eingesetzte Volumen an peripherem Blut berechnet.

## Patentansprüche

1. Verfahren zum quantitativen Nachweis vitaler epithelialer Tumorzellen in einer bestimmten Menge einer Körperflüssigkeit mit folgenden Schritten:
a) Markieren der vitalen epithelialen Tumorzellen durch Zugabe von gegen humanes epitheliales Antigen gerichteten Antikörpern, die an magnetische Partikel gebunden sind, zu der Körperflüssigkeit, wobei das humane epitheliale Antigen ein Antigen ist, welches durch den monoklonalen Antikörper HEA 125 erkannt wird,
b) Markieren der vitalen epithelialen Tumorzellen durch Zugabe von gegen das humane epitheliale Antigen gerichteten Antikörpern, die an ein Fluorochrom gebunden sind, zu der Körperflüssigkeit,
c) Magnetische Anreicherung der vitalen epithelialen Tumorzellen,
d) Immobilisieren der so erhaltenen Suspension auf einem Trägermaterial durch selbständiges Anhaften der vitalen Tumorzellen,
e) Erfassen der vitalen epithelialen Tumorzellen mittels Laser-Scanning-Cytometrie und Berechnen der Anzahl dieser Zellen in bezug auf die Menge der Körperflüssigkeit.

2. Verfahren nach Anspruch 1, wobei mittels Laser-Scanning-Cytometrie die maximale Fluoreszenzintensität und/oder die Gesamtfluoreszenz pro Zelle bestimmt wird.

3. Verfahren nach einem der vorstehenden Ansprüche, wobei die Hintergrundfluoreszenz dynamisch bestimmt wird, so daß für jede Zelle äquivalente Fluoreszenzwerte erhalten werden.

4. Verfahren nach einem der vorstehenden Ansprüche, wobei die magnetische Anreicherung der vitalen epithelialen Tumorzellen vor der Markierung der vitalen epithelialen Tumorzellen durch Zugabe von gegen das humane epitheliale Antigen gerichteten Antikörpern, die an ein Fluorochrom gebunden sind, zu der Körperflüssigkeit erfolgt.

5. Verfahren nach einem der vorstehenden Ansprüche, wobei die magnetische Anreicherung der vitalen epithelialen Tumorzellen nach der Markierung der vitalen epithelialen Tumorzellen durch Zugabe von gegen das humane epitheliale Antigen gerichteten Antikörpern, die an ein Fluorochrom gebunden sind, zu der Körperflüssigkeit erfolgt.

6. Verfahren nach einem der vorstehenden Ansprüche, wobei die Körperflüssigkeit peripheres Blut ist, welches lysiert und zentrifugiert wird, bevor die vitalen epithelialen Tumorzellen mit Antikörpern markiert werden, wobei der Überstand abgetrennt und verworfen wird.

7. Verfahren nach einem der vorstehenden Ansprüche, wobei zu der Körperflüssigkeit vor dem Markieren der vitalen epithelialen Tumorzellen ein FcR-Blockierungsreagenz zugesetzt wird.

8. Verfahren nach einem der vorstehenden Ansprüche, wobei zur Markierung der vitalen epithelialen Tumorzellen mit gegen das humane epitheliale Antigen gerichteten Antikörpern von der Maus stammende gegen das humane epitheliale Antigen gerichtete Antikörper verwendet werden.

9. Verfahren nach einem der vorstehenden Ansprüche, wobei als Fluorochrom Fluoresceinisothiocyanat (FITC) eingesetzt wird.

10. Verfahren nach einem der vorstehenden Ansprüche, wobei die Körperflüssigkeit aus der folgenden Gruppe ausgewählt wird: Blut, Knochenmark, Knochenmark-Aspirat, Transsudat, Exsudat, Lymphe, Apheresat, Aszites, Urin, Speichel und Drainageflüssigkeiten aus Wundsekreten.

## Claims

1. Method for quantitative detection of vital epithelial tumour cells in a defined quantity of a body fluid, comprising the following steps:
a) labelling the vital epithelial tumour cells by addition, to the body fluid, of antibodies directed against human epithelial antigen which antibodies are bound to magnetic particles, wherein said human epithelial antigen is an antigen that is recognised by the monoclonal antibody HEA 125.
b) labelling the vital epithelial tumour cells by addition, to the body fluid, of antibodies directed against said human epithelial antigen which antibodies are bound to a fluorochrome,
c) magnetically enriching the vital epithelial tumour cells,
d) immobilising the suspension so obtained on a support material by independent adhesion of the vital tumour cells,
e) recording the vital epithelial tumour cells by means of laser scanning cytometry and calculating the number of these cells in relation to the quantity of body fluid.

2. Method according to Claim 1, in which laser scanning cytometry is carried out to determine the maximum fluorescence intensity and/or the total fluorescence per cell.

3. Method according to one of the preceding claims, in which the background fluorescence is dynamically determined so that equivalent fluorescence values are obtained for each cell.

4. Method according to one of the preceding claims, in which the magnetic enrichment of the vital epithelial tumour cells takes place prior to the labelling of the vital epithelial tumour cells by addition, to the body fluid, of antibodies directed against said human epithelial antigen which antibodies are bound to a fluorochrome.

5. Method according to one of the preceding claims, in which the magnetic enrichment of the vital epithelial tumour cells takes place after the labelling of the vital epithelial tumour cells by addition, to the body fluid, of antibodies directed against said human epithelial antigen which antibodies are bound to a fluorochrome.

6. Method according to one of the preceding claims, in which, prior to the labelling of the vital epithelial tumour cells with antibodies, the body fluid is peripheral blood which is lyzed and centrifuged and the supernatant separated off and discarded.

7. Method according to one of the preceding claims, in which an FcR blocking reagent is added to the body fluid prior to the labelling of the vital epithelial tumour cells.

8. Method according to one of the preceding claims, in which antibodies from mice directed against said human epithelial antigen are used to label the vital epithelial tumour cells with antibodies directed against said human epithelial antigen.

9. Method according to one of the preceding claims, in which fluorescein isothiocyanate (FITC) is used as fluorochrome.

10. Method according to one of the preceding claims, in which the body fluid is chosen from the following group: blood, bone marrow, bone marrow aspirate, transudate, exudate, lymph, apheresis fluid, ascites, urine, saliva, and drainage fluids from wound secretions.

## Revendications

1. Procédé pour la mise en évidence ou la détermination quantitative de cellules tumorales épithéliales vivantes dans une quantité déterminée d'un fluide corporel, comportant les étapes suivantes :
a) marquage des cellules tumorales épithéliales vivantes par ajout, dans le fluide corporel, d'anticorps dirigés contre un antigène épithélial humain et qui sont liés à des particules, magnétiques, l'antigène épithélial humain étant un antigène qui est reconnu par l'anticorps monoclonal HEA 125,
b) marquage des cellules tumorales épithéliales vivantes par ajout, dans le fluide corporel, d'anticorps dirigés contre l'antigène épithélial humain et qui sont liés à un fluorochrome,
c) enrichissement magnétique des cellules tumorales épithéliales vivantes,
d) immobilisation de la suspension ainsi obtenue sur un matériau de support par adhésion indépendante ou autonome des cellules tumorales vivantes,
e) acquisition des cellules tumorales épithéliales vivantes au moyen d'une cytométrie à balayage laser et calcul du nombre de ces cellules par rapport à la quantité du fluide corporel.

2. Procédé selon la revendication 1, dans lequel on détermine, au moyen d'une cytométrie à balayage laser, l'intensité de fluorescence maximale et/ou la fluorescence totale par cellule.

3. Procédé selon l'une des revendications précédentes, dans lequel la fluorescence de fond est déterminée de façon dynamique, si bien que l'on obtient pour chaque cellule des valeurs de fluorescence équivalentes.

4. Procédé selon l'une des revendications précédentes, dans lequel l'enrichissement magnétique des cellules tumorales épithéliales vivantes se produit avant le marquage des cellules tumorales épithéliales vivantes par ajout, dans le fluide corporel, d'anticorps dirigés contre l'antigène épithélial humain et qui sont liés à un fluorochrome.

5. Procédé selon l'une des revendications précédentes, dans lequel l'enrichissement magnétique des cellules tumorales épithéliales vivantes se produit après le marquage des cellules tumorales épithéliales vivantes par ajout, dans le fluide corporel, d'anticorps dirigés contre l'antigène épithélial humain et qui sont liés à un fluorochrome.

6. Procédé selon l'une des revendications précédentes, dans lequel le fluide corporel est du sang périphérique qui est lysé et centrifugé avant que les cellules tumorales épithéliales vivantes soient marquées avec les anticorps, le surnageant étant séparé et jeté.

7. Procédé selon l'une des revendications précédentes, dans lequel on ajoute au fluide corporel avant le marquage des cellules tumorales épithéliales vivantes un réactif de blocage des FcR.

8. Procédé selon l'une des revendications précédentes, dans lequel, pour marquer les cellules tumorales épithéliales vivantes avec des anticorps dirigés contre l'antigène épithélial humain, on utilise des anticorps provenant de souris et dirigés contre l'antigène épithélial humain.

9. Procédé selon l'une des revendications précédentes, dans lequel on utilise comme fluorochrome de l'isothiocyanate de fluorescéine (FITC).

10. Procédé selon l'une des revendications précédentes, dans lequel le fluide corporel est sélectionné dans le groupe suivant : sang, moelle osseuse, aspirat de moelle osseuse, transsudat, exsudat, lymphe, produit d'aphérèse, ascite, urine, salive et fluides de drainage de sécrétions de plaies.
